(19) Europäisches Patentamt
European Patent Office
Office européen des brevets

(11) Publication number: **0 480 217 A2**

(12) **EUROPEAN PATENT APPLICATION**

(21) Application number: **91115991.1**

(51) Int. Cl.[5]: **C07C 22/00**, C09K 19/30

(22) Date of filing: **20.09.91**

(30) Priority: **11.10.90 EP 90119484**

(43) Date of publication of application: **15.04.92 Bulletin 92/16**

(84) Designated Contracting States: **DE GB**

(71) Applicant: **MERCK PATENT GESELLSCHAFT MIT BESCHRÄNKTER HAFTUNG**
**Frankfurter Strasse 250 Postfach 4119**
**W-6100 Darmstadt(DE)**

(72) Inventor: **Reiffenrath, Volker**
**Jahnstrasse 18**
**W-6101 Rossdorf(DE)**
Inventor: **Plach, Herbert, Dr.**
**Wingertsbergstrasse 5**
**W-6100 Darmstadt(DE)**

(54) **Mesogenic compounds.**

(57) The invention concerns mesogenic compounds of formula I

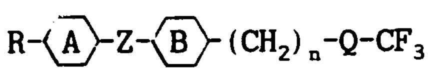

I

wherein

- R is a unsubstituted, a mono cyano or trifluoromethyl substituted or a mono-, oligo- or polyhalogeno-substituted alkyl or alkenyl residue having 1 to 15 carbon atoms, or such a residue wherein one or more $CH_2$ groups are each independently replaced by -O-, -CO-, -CO-O-, -O-CO- or -O-CO-O- with the proviso that oxygen atoms are not directly attached to each other,

the rings A and B independently are trans-1,4-cyclohexylene groups or one of the rings A and B may also be a trans, trans-4,4'-bicyclohexyl group,

- Z is $-CH_2CH_2CH_2CH_2-$, $-CH_2CH_2-$ or a single bond,
- Q is -C≡C- or -CH=CH(trans)- and
- n is zero or an integer from 1 to 10.

EP 0 480 217 A2

The invention relates to mesogenic compounds of formula I

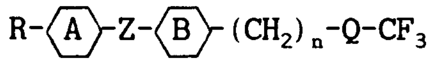

I

wherein

R is a unsubstituted, a mono cyano or trifluoromethyl substituted or a mono-, oligo- or polyhalogeno-substituted alkyl or alkenyl residue having 1 to 15 carbon atoms, or such a residue wherein one or more $CH_2$ groups are each independently replaced by -O-, -CO-, -CO-O-, -O-CO- or -O-CO-O- with the proviso that oxygen atoms are not directly attached to each other,

the rings A and B independently are trans-1,4-cyclohexylene groups or one of the rings A and B may also be a trans, trans-4,4'-bicyclohexyl group,

Z is $-CH_2CH_2CH_2CH_2-$, $-CH_2CH_2-$ or a single bond,

Q is -C≡C- or -CH = CH(trans)- and

n is zero or an integer from 1 to 10.

The invention further relates to liquid crystalline media comprising at least two liquid crystalline components at least one of which being a compound of formula I, further to electro-optical systems containing such media and to the use of the compounds of formula I as components of liquid crystalline media.

For simplicity, in the following text Cyc is a trans-1,4-cyclohexylene group.

The compounds of the formula I can be used as components of liquid crystal media, in particular for electrooptical systems and liquid crystal displays, based on the principle of the twisted nematic cell including cells with twist angles different from 90° (e.g. STN, SBE or OMI), the guest-host effect, the effect of deformation of aligned phases or the effect of dynamic scattering. Due to their superior stability versus heat and light the compounds of formula I are especially suited for active matrix addressed displays.

The invention was based on the object of discovering new liquid crystal or mesogenic compounds being suited as components of liquid crystal media and especially exhibiting a broad nematic mesophase range, a rather low optical birefringence and viscosity, a high stability versus chemicals and thermal and electromagnetical energy, especially versus UV-light and light of the short wavelength range of the visible spectrum, advantageous values for the elastic constants, the dielectric anisotropy, a good electrooptical and thermooptical response and a good miscibility with other, including known liquid crystal compounds.

It has been found, that the compounds of formula I are outstandingly suitable as components of liquid crystalline media. In particular, liquid crystalline media having a broad nematic mesophase range, a very low birefringence, a rather low viscosity and a high stability versus chemicals, heat and light and advantageous values of the dielectric anisotropy can be prepared with the aid of these compounds.

By providing the compounds of the formula I, the range of liquid crystal substances which are suitable under various technological aspects for the preparation of liquid crystal media is moreover quite generally considerable extended.

The compounds of the formula I have a wide range of application. Depending on the choice of the substitutents, these compounds can be used as base materials of which liquid crystal media are predominantly composed; however, compounds of the formula I can also be added to liquid crystal base materials of other classes of compounds, for example in order to vary the elastic constants, the dielectric and/or optical anisotropy and/or the viscosity and/or the phase range of such dielectric.

The compounds of the formula I are furthermore suitable as intermediates for the preparation of other substances which can be used as constituents of liquid crystal dielectrics.

The compounds of the formula I are colourless in the pure state and form liquid crystal mesophases in a temperature range which is favourably located for electrooptical use.

They are very stable towards chemicals, heat and light and exhibit advantageous values for the optical and dielectric anisotropy, the elastic constants, the phase range and the viscosity and they exhibit a good electrooptical and thermooptical response and a good miscibility with other liquid crystal compounds.

The invention thus relates to compounds of the formula I and to the use of the compounds of the formula I as components of liquid crystalline media. The invention furthermore relates to liquid crystalline media containing at least one compound of the formula I and to liquid crystal displays containing such media.

Above and below R, the rings A and B, Z, Q and n have the meaning given, unless expressly indicated otherwise.

The compounds of the formula I accordingly include compounds with a ω-$CF_3$-alkenyl and a ω-$CF_3$-alkinyl group according to formulae Ia and Ib:

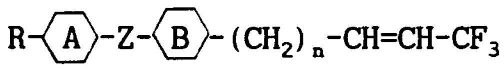

Ia

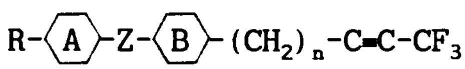

Ib

these formulae comprise the following preferred binuclear and trinuclear core structures

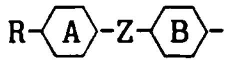

of the formulae 1 to 6:

R-Cyc-Cyc- 1

R-Cyc-$CH_2CH_2$-Cyc- 2

R-Cyc-$CH_2CH_2CH_2CH_2$-Cyc- 3

R-Cyc-Cyc-Cyc- 4

R-Cyc-$CH_2CH_2$-Cyc-Cyc- 5

R-Cyc-Cyc-$CH_2CH_2$-Cyc- 6

In the compounds of the formulae Ia and Ib n is preferably 0-7 and, in particular, 0.

In the compounds of formulae Ia and Ib Z preferably denotes a single bond.

The elastic constants of the compounds of the formule Ia and Ib can be influenced and optimized by varying the length of the side chain with the terminal $CF_3$ group; thus it is possible, for example, to provide compounds, resp. liquid crystalline media containing these compounds exhibiting improved values of the threshold voltage and/or the steepness of the electrooptical characteristic line.

The terminal substituent R in the compounds of formula I preferably contains 1-13 C atoms and in particular 1-11 C atoms. It is also possible for one or more CH groups in R to be replaced. Preferably, only one $CH_2$ group is replaced by -O-, -CO-, -OCO-, -COO- or -OCOO-, in particular by -O-, -CO-, -OCO- or -COO-.

If R is an alkyl radical in which one ("alkoxy" or "oxaalkyl") or two ("alkoxyalkoxy" or "dioxaalkyl") non-adjacent $CH_2$ groups can also be replaced by 0 atoms, it can be straight-chain or branched. Preferably, it is straight-chain, has 2, 3, 4, 5, 6 or 7 C atoms and is preferably ethyl, propyl, butyl, pentyl, hexyl, heptyl, ethoxy, propoxy, butoxy, pentoxy, hexoxy or heptoxy, or furthermore methyl, octyl, nonyl, decyl, undecyl, dodecyl, tridecyl, tetradecyl, pentadecyl, methoxy, octoxy, nonoxy, decoxy, undecoxy, dodecoxy, tridecoxy, tetradecoxy or pentadecoxy.

Oxaalkyl is preferably straight-chain 2-oxapropyl (= methoxymethyl), 2- (= ethoxymethyl) or 3-oxabutyl (= 2-methoxymethyl, 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, 2-, 3-, 4-, 5-, 6-, 7-, 8- or 9-oxadecyl, 1,3-dioxabutyl (=methoxymethoxy), 1,3-, 1,4-, 2,4-dioxapentyl, 1,3-, 1,4-, 1,5-, 2,4-, 2,5- or 3,5-dioxahexyl, 1,3-, 1,4-, 1,5-, 1,6-, 2,4-, 2,5-, 2,6-, 3,5-, 3,6- or 4,6-dioxaheptyl.

If R is an alkyl radical in which a $CH_2$ group is replaced by -O-CO or -CO-O-, this can be straight-chain or branched. Preferably, it is straight-chain and has 2 to 6 C atoms. It is accordingly in particular acetyloxy, propionyloxy, butyryloxy, pentanoyloxy, hexanoyloxy, acetyloxymethyl, propionyloxymethyl, butyryloxymethyl, pentanoyloxymethyl, 2-acetyloxymethyl, 2-propionyloxyethyl, 2-butyryloxyethyl, 3-acetyloxypropyl, 3-propionyloxyethyl, 2-butyryloxyethyl, 3-acetyloxypropyl, 3-propionyloxypropyl, 4-acetyloxybutyl, methoxycarbonyl, ethoxycarbonyl, propoxycarbonyl, butoxycarbonyl, pentoxycarbonyl, methoxycarbonylmethyl,

ethoxycarbonylmethyl, propoxycarbonylmethyl, butoxycarbonylmethyl, 2-(methoxycarbonyl)ethyl, 2-(ethoxycarbonyl)ethyl, 2-(propoxycarbonyl)ethyl, 3-(methoxycarbonyl)propyl, 3-(ethoxycarbonyl)propyl or 4-(methoxycarbonyl)butyl.

If R is a mono-, oligo- or polyhalogeno substituted alkyl radical, this can be straight-chain or branched. Preferably it is straight-chain, has 3-12 C atoms and is mono-, di-, tri- or tetrahalogenated, particularly preferably with F and/or Cl. R preferably is 2-fluoroethyl, 2,2-difluoroethyl, 2,2,2-trifluoroethyl, 2-fluoropropyl, 3,3,3-trifluoropropyl, 2,3-difluoropentyl, 2-chlorobutyl, 4,4,4-trifluorobutyl, 4,5-difluoropentyl, 5-fluorpentyl, 5,5-difluoropentyl, 5,5,5-trifluoropentyl, 5,5,5-trifluoro-3-fluoro-pentyl, 6,6,6-trifluorohexyl, 6-fluorohexyl, 6-chlorohexyl, 6,6-difluorohexyl, 6-fluorohexyl, 6-chlorohexyl, 6,6-difluorohexyl, 7,7,7-trifluoroheptyl, 3,5-difluoroheptyl, 7-chloroheptyl, 7,7-dichloroheptyl, 8,8-difluorooctyl, 8,8,8-trifluorooctyl, 8-chlorooctyl and 9,9,9-trifluorononyl.

"Symmetrical" compounds exhibiting identical terminal groups are furthermore preferred.

Amongst the compounds of the formulae Ia and Ib, those in which at least one of the radicals contained therein has one of the preferred meanings given are preferred.

The compounds according to formula I are chemically very stable and they exhibit a high temperature and UV stability superior to that of cyano compounds for example and simultaneously have surprisingly high clearing points and low viscosity.

The compounds of formula I are further characterized by a relatively high or even high dielectric anisotropy. These compounds are therefore very well suited as components of liquid crystalline media which are used in displays based on the principle of the twisted nematic cell. The term twisted nematic cell is used here in a wide sense and comprises cells with twist angles ranging from 0° to 360° such as, for example, TN (twisted nematic), supertwisted nematic (STN), supertwisted birefringence (SBE) or low twisted nematic (LTN, $\beta < 90°$) cells. Due to their high stability the $\omega$-$CF_3$-alkenyl and $\omega$-$CF_3$-alkinyl compounds are especially preferred for active matrix addressed displays. It is also possible, however, for these compounds to be used in displays based on the guest-host effect, the effect of deformation of aligned phases or the effect of dynamic scattering.

These compounds are well suited, for example, for liquid crystalline media for TN displays being operated under the so-called first-minimum condition and especially for low-$\Delta n$-TFT applications.

The threshold voltage is further influenced by the elastic constants of the liquid crystalline compounds. The threshold voltage of a TN structure, for example, is given via

$$V_{th} = \pi \left(\frac{k'}{\varepsilon_o \, \Delta\varepsilon}\right)^{1/2}$$

wherein $\epsilon_o$ is the dielectric constant of the vacuum, $\Delta\epsilon$ is the dielectric anisotropy of the liquid crystal and k' denotes an elastic constant primarily dependent on the splay elastic constant $k_{11}$. The compounds of formula I are advantageously used for low threshold voltage TN displays. The elastic constants of the compounds can be modified to a great extent by varying the chain length of the $\omega$-$CF_3$ group

$-(CH_2)_n-Q-CF_3$

Thus it is possible to optimize the elastic properties of the $\omega$-$CF_3$-alkenyl and the $\omega$-$CF_3$-alkinyl compounds not only for TN applications which were merely considered as an example but also for other applications.

Compounds of the formula I wherein one terminal group is a $\omega$-$CF_3$-alkenyl or a $\omega$-$CF_3$-alkinyl group and the other terminal group is a straight-chain or not more than a singly branched alkyl group with 1-12, in particular 2-10 C atoms are especially preferred. In the compounds of formula I n preferably denotes 0-8, particularly 0-6 and is preferably even. Compounds exhibiting an odd number for n are secondly preferred.

Compounds with a $\omega$-$CF_3$-alkenyl group are the (E)-isomers

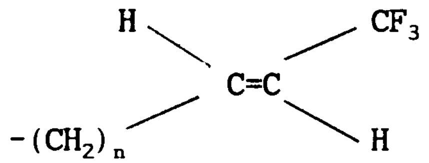

(E)-isomer

because the (Z)-isomers are clearly inferior in their overall LC properties.

Compounds with a $\omega$-$CF_3$-alkinyl group are often characterized by especially advantageous values of the elastic constants, the birefringence and/or the phase transition temperatures and these compounds are therefore preferred.

Especially preferred are $\omega$-$CF_3$-alkinyl terminal groups with the follow-ing values for n: 0 or 2.

The compounds of the formula I are prepared by methods which are known per se, such as are described in the literature (for example in the standard works such as Houben-Weyl, Methoden der Organischen Chemie (Methods of Organic Chemistry), Georg-Thieme-Verlag, Stuttgart), and in particular under reaction conditions which are known and suitable for the reactions mentioned. In these reactions, it is also possible to utilize variants which are known per se and are not mentioned here in more detail.

If desired, the starting substances can also be formed in situ such that they are not isolated from the reaction mixture but are immediately reacted further to give the compounds of the formula I.

Compounds of formula I with an $\omega$-$CF_3$-alkenyl group can be obtained for example via a Wittig reaction according to the following scheme:

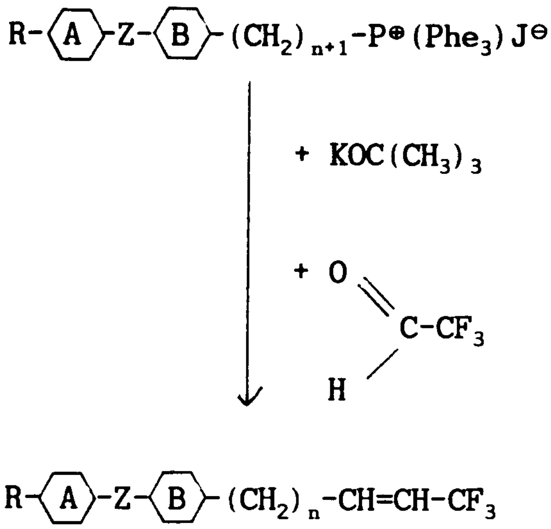


Compounds of formula I with a $\omega$-$CF_3$-alkinyl group may be prepared by a number of generally applicable routes, for example, according to the following scheme:

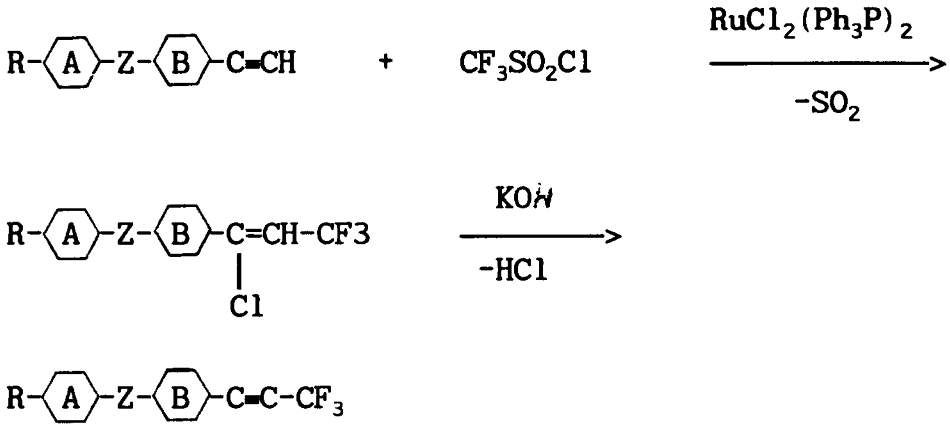


Compounds of formula I with a ω-$CF_3$-alkinyl group can further be obtained, for example, via reacting a ω-$CF_3$-alkenyl compound of formula I with bromine with subsequent elimination of HBr.

Compounds of formula I with a ω-$CF_3$-alkenyl or ω-$CF_3$-alkinyl group can further preferably be obtained by reacting

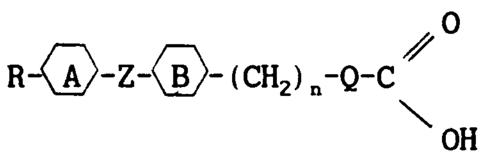

with sulfur tetrafluoride or diethylamino sulfur trifluoride (DAST).

It is further possible to convert appropriate $CF_3$-Q-$(CH_2)_n$-terminal substituted precursors to the compounds of formula I via a metal-catalyzed cross-coupling reaction as is described, for example, in E. Poetsch, Kontakte (Darmstadt, 1988 (2), p. 15ff).

Other methods of preparing compounds of formula I are apparent to those skilled in the art.

The liquid crystal media according to the invention preferably contain 2 to 40, in particular 4 to 30, components as further constituents in addition to one ore more compounds according to the invention. These media especially preferably contain 7 to 25 components in addition to one ore more compounds according to the invention. These other constituents are preferably chosen from the nematic or nematogenic (monotropic or isotropic) substances, in particular substances from the classes of azoxybenzenes, benzylideneanilines, biphenyls, terphenyls, phenyl or cyclohexyl benzoates, phenyl or cyclohexylcyclohexanecarboxylates, phenyl or cyclohexyl esters of cyclohexylbenzoid acid, phenyl or cyclohexyl esters of cyclohexylbenzoic acid, phenyl or cyclohexyl esters of cyclohexylcyclohexanecarboxylic acid, phenylcyclohexanes, cyclohexylbiphenyls, phenylcyclohexylcyclohexanes, cyclohexylcyclohexanes, cyclohexylcyclohexenes, cyclohexylcyclohexylcyclohexenes, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexyldioxanes, phenyl- or cyclohexyl-1,3-dithianes, 1,2-diphenylethanes, 1,2-dicyclohexylethanes, 1-phenyl-2-cyclohexylethanes, 1-cyclohexyl-2-(4-phenyl-cyclohexyl)-ethanes, 1-cyclohexyl-2-biphenylylethanes, 1-phenyl-2-cyclohexylphenylethanes, optionally halogenated stilbenes, benzyl phenyl ethers, tolans and substituted cinnamic acids. The 1,4-phenylene groups in these compounds can also be fluorinated.

The most important compounds which are possible further constituents of media according to the invention can be characterized by the formulae 1, 2, 3, 4 and 5:

R'-L-E-R" 1

R'-L-COO-E-R" 2

R'-L-OOC-E-R" 3

R'-L-$CH_2CH_2$-E-R" 4

R'-L-C≡C-E-R''     5

In the formulae 1, 2, 3, 4 and 5, L and E, which can be identical or different, in each case independently of one another are a bivalent radical from the group comprising -Phe-, -Cyc-, -Phe-Phe-, -Phe-Cyc-, -Cyc-Cyc-, -Pyr-, -Dio-, -G-Phe- and -G-Cyc- and mirror images thereof, wherein Phe is 1,4-phenylene which is unsubstituted or substituted by fluorine, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)-ethyl, pyrimidine-2,5-diyl, pyridine-2,5-diyl or 1,3-dioxane-2,5-diyl.

Preferably, one of the radicals L and E is Cyc, Phe or Pyr. E is preferably Cyc, Phe or Phe-Cyc. The media according to the invention preferably contain one ore more components chosen from the compounds of the formulae 1, 2, 3, 4 and 5, wherein one of the radicals L and E is chosen from the group comprising Cyc, Phe and Pyr and the other radical is chosen from the group comprising -Phe-Phe-, -Phe-Cyc-, -Cyc-Cyc-, -G-Phe- and -G-Cyc-, and if appropriate one ore more components chosen from the compounds of the formulae 1, 2, 3, 4 and 5 wherein the radicals L and E are chosen from the group comprising -Phe-Cyc-, -Cyc-Cyc-, -G-Phe- and -G-Cyc-.

R' and R'' in the compounds of the part formulae I1, 2a, 3a, 4a and 5a in each case independently of one another are alkyl, alkenyl, alkoxy, alkenyloxy or alkanoyloxy having up to 8 carbon atoms. In most of these compounds, R' and R'' differ from one another, one of these radicals usually being alkyl or alkenyl. In the compounds of the part formulae 1b, 2b, 3b, 4b and 5b, R'' is -CN, $-CF_3$, F, Cl or -NCS; R here has the meaning given in the case of the compounds of the part formulae 1a to 5a, and is preferably alkyl or alkenyl. However, other variants of the envisaged substituents in the compounds of the formulae 1, 2, 3, 4 and 5 can also be used.

Many such substances or mixtures thereof are commercially available. All these substances are obtainable by methods which are known from the literature or by analogous methods.

The media according to the invention preferably also contain, in addition to components from the group of compounds 1a, 2a, 3a, 4a and 5a (group 1), components from the group of compounds 1b, 2b, 3b, 4b and 5b (group 2), the propertions of which are preferably as follows:

Group 1:     20 to 90 %, in particular 30 to 90 %,
Group 2:     10 to 80 %, in particular 10 to 50 %,

the sum of the proportion of the compounds according to the invention and the compounds from groups 1 and 2 being up to 100 %.

The media according to the invention preferably contain 1 to 50 %, particularly preferably 5 to 30 %, of compounds according to the invention. Media containing more than 40 %, in particular 45 to 90 %, of compounds according to the invention are furthermore preferred. The media preferably contain three, four or five compounds according to the invention.

The media according to the invention are prepared in a manner which is customary per se. As a rule, the components are dissolved in one another, preferably at elevated temperature. The liquid crystal phases according to the invention can be modified by suitable additives so that they can be used in all the types of liquid crystal display elements disclosed to date. Such additives are known to the expert and are described in detail in the literature (H. Kelker/ R. Hatz, Handbook of Liquid Crystals, Verlag Chemie, Weinheim, 1980). For example, it is possible to add pleochroic dyestuffs to prepare coloured guest-host systems or substances to change the dielectric anisotropy, the viscosity and/or the orientation of the nematic phases.

The liquid crystalline media according to the invention may be nematic or smectic; preferably, however, they are nematic with the term nematic media embracing also chiral nematic = cholesteric media.

The following examples are intended to illustrate the invention without limiting it. mp. = melting point, cp. = clearing point. Percentage data above and below are percentages by weight; all the temperatures are stated in degrees Celsius. "Customary working up" means: water is added, the mixture is extracted with methylene chloride, the organic phase is separated off, dried and evaporated and the product is purified by crystallization and/or chromatography.

Further abbreviations have the following meanings: C: crystalline-solid state, S: smectic phase (the index identifies the phase type), N: nematic state, Ch: cholesteric state, I: isotropic phase. The number between two symbols indicates the transition temperature in degrees Celsius.

Examples of substances

Example 1

0.05 mol of [trans-4-(trans-4-pentylcyclohexyl)-cyclohexyl]-methyl-triphenyl phosphonium iodide are dissolved in 100 ml tetrahydrofuran (THF) and 0.05 mol of 2,2,2-trifluoroacetaldehyde are added. To this mixture a solution of potassium tert.-butylate in THF is added dropwise at a temperature of 0-5 °C and the resulting mixture is stirred for one hour at room temperature. Then water and diethylether are added and trans-,trans-4-pentyl-4'-(3,3,3-trifluoroprop-1-enyl)-cyclohexylcyclohexane is extracted and purified by chromatography.

The purified (Z)-isomer shows C 59 N (57) I, $\Delta\epsilon$ = +5.8, $\Delta n$ = 0.053.

The following compounds are prepared analogously:

trans-,trans-4-ethyl-4'-(3,3,3-trifluorprop-1-enyl)-cyclohexylcyclohexane
trans-,trans-4-propyl-4'-(3,3,3-trifluorprop-1-enyl)-cyclohexylcyclohexane C 60 $S_B$ 61 I, $\Delta\epsilon$ = +6.2, $\Delta n$ = 0.051
trans-,trans-4-butyl-4'-(3,3,3-trifluorprop-1-enyl)-cyclohexylcyclohexane
trans-,trans-4-hexyl-4'-(3,3,3-trifluorprop-1-enyl)-cyclohexylcyclohexane
trans-,trans-4-heptyl-4'-(3,3,3-trifluorprop-1-enyl)-cyclohexylcyclohexane
trans-,trans-4-octyl-4'-(3,3,3-trifluorprop-1-enyl)-cyclohexylcyclohexane
trans-,trans-4-nonyl-4'-(3,3,3-trifluorprop-1-enyl)-cyclohexylcyclohexane
trans-,trans-4-decyl-4'-(3,3,3-trifluorprop-1-enyl)-cyclohexylcyclohexane

Example 2

A sealed mixture of 0.05 mol of 1-[trans-4-[trans-4-n-pentylcyclohexyl)-cyclohexyl]-ethine, 0.05 mol of trifluoromethylsulfonylchloride, 0.5 mmol of dichlorobis(triphenylphosphin)-ruthenium-(II) and 50 ml of toluene is heated at 120 °C for 16 hours. After extractive working-up the intermediate chloro compound is purified by chromatography. After elimination of HCL from this intermediate produt (KOH/tert.-butanole, 50 °C, 2 hours) trans,trans-4-n-pentyl-4'-(3,3,3-trifluoroprop-1-inyl)-cyclohexyl-cyclohexane is obtained.

The following compounds are prepared analogously:

trans,trans-4-ethyl-4'-(3,3,3-trifluoroprop-1-inyl)-cyclohexylcyclohexane
trans,trans-4-propyl-4'-(3,3,3-trifluoroprop-1-inyl)-cyclohexylcyclohexane
trans,trans-4-butyl-4'-(3,3,3-trifluoroprop-1-inyl)-cyclohexylcyclohexane
trans,trans-4-hexyl-4'-(3,3,3-trifluoroprop-1-inyl)-cyclohexylcyclohexane
trans,trans-4-heptyl-4'-(3,3,3-trifluoroprop-1-inyl)-cyclohexylcyclohexane
trans,trans-4-octyl-4'-(3,3,3-trifluoroprop-1-inyl)-cyclohexylcyclohexane
trans,trans-4-nonyl-4'-(3,3,3-trifluoroprop-1-inyl)-cyclohexylcyclohexane
trans,trans-4-decyl-4'-(3,3,3-trifluoroprop-1-inyl)-cyclohexylcyclohexane

## Claims

**1.** Mesogenic compounds of formula I

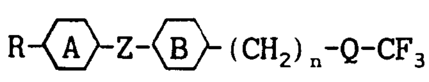

I

wherein

R is a unsubstituted, a mono cyano or trifluoromethyl substituted or a mono-, oligo- or polyhalogeno-substituted alkyl or alkenyl residue having 1 to 15 carbon atoms, or such a residue wherein one or more $CH_2$ groups are each independently replaced by -O-, -CO-, -CO-O-, -O-CO- or -O-CO-O- with the proviso that oxygen atoms are not directly attached to each other,

the rings A and B independently are trans-1,4-cyclohexylene groups or one of the rings A and B may also be a trans, trans-4,4'-bicyclohexyl group,

Z is $-CH_2CH_2CH_2CH_2-$, $-CH_2CH_2-$ or a single bond,

Q is -C≡C- or -CH=CH(trans)- and

n is zero or an integer from 1 to 10.

**2.** Liquid crystalline medium with at least two liquid crystalline components one component being a compound of formula I.

**3.** Electro-optical system containing a liquid crystalline medium according to claim 2.

**4.** Use of the compounds of the formula I according to claim 1 as components of liquid crystalline media.